# EUROPEAN PATENT SPECIFICATION

(11) **EP 0 731 705 B1**
(45) Date of publication and mention of the grant of the patent: **07.11.2001**
(21) Application number: 95903316.8
(22) Date of filing: 02.12.1994
(51) Int. Cl.: A61K 31/80

(54) **USE OF DIMETICONE FOR TREATING CONSTIPATION**
VERWENDUNG VON DIMETICON ZUR BEHANDLUNG DER KONSTIPATION
UTILISATION DU DIMETICONE DANS LE TRAITEMENT DE LA CONSTIPATION

(30) Priority: 02.12.1993 DE 4341165
(43) Date of publication of application: 18.09.1996
(73) Proprietor: Schmidt, Alfred, 22529 Hamburg (DE); Upmeyer, Hans-Jürgen, 80796 München (DE)
(72) Inventor: Schmidt, Alfred, 22529 Hamburg (DE); Upmeyer, Hans-Jürgen, 80796 München (DE)
(74) Representative: VOSSIUS & PARTNER
(86) International application number: EP9404024
(87) International publication number: WO9515170

(56) References cited:
- DE-A- 3 807 712
- FR-M- 5 549
- THERAPIEWOCHE, vol. 25,no. 12, 1975 pages 1509-12, 'Zur Therapie der chronischen Obstipation: Behandlung mit Gillazym'
- MED.J.AUST., vol. 150,no. 11, 1989 page 667 'Cisapride for severe non-ulcer dyspepsia, pseudoobstruction and constipation'
- J.INDIANA MED.ASS., vol. 66,no. 12, 1973 pages 1083-4, 'Constipation in geriatric patients'
- RADIOLOGE, vol. 30,no. 1, 1990 pages 34-8, 'Vergleichende Studie zur Vorbereitung der Doppelkontrastuntersuchung des Kolons: Prepacol versus Rizinuskapseln mit Reinigungseinlauf'

## Description

Constipation means a delay in the discharge of dry and hard feces. It is caused either by a delayed passage of the stomach contents through the intestine or a disorder of the evacuation reflex. Possible reasons for the delayed passage through the intestine are dietetic factors, changes in the intestinal walls or a functional or organic disorder of the nervous system. Also, pharmaceuticals may have a constipating effect. A defect in the defecation mechanism is found in disorders of the anal channel, in the case of loss of the rectal dilation reflex or a weakness in the abdominal muscles used to apply abdominal pressure. Patients complain about symptoms such as a sensation of pressure, flatulence, and pains during the discharge of feces.

Constipation is treated with laxatives which on the one hand soften the hard feces and on the other hand prompt defecation.

Laxatives either act as lubricants, fillers or swelling agents, have saline or osmotic properties, or produce an anti-absorptive or secretagogue effect. The substances of the afore-mentioned groups are combined in commercial preparations in many ways.

When used in long-term therapy, almost all laxatives, except for the swelling agents, increasingly cause disorders of the electrolyte metabolism and in particular cause a potassium loss, which in turn increases constipation. The sodium loss can be so high as to cause a secondary hyperaldesteronism. The potassium losses so caused may lead to a reduced intestine motility and may increase constipation. Moreover, hypokalaemias are above all dangerous when used in therapy together with cardiac glycosides. Concomitant calcium losses can promote the occurrence of osteoporosis, especially in women.

The administration of a swelling agent involves the danger that the stomach contents might stick together, from which the danger of an ileus ensues.

Moreover, many laxatives are resorbed, with the result that they can have a large number of systemic side effects.

Hence, there has been a need for an effective agent for treating constipation which does not produce the afore-mentioned side effects and consequently lends itself for use in problem patients, for instance children, immobile or geriatric patients and women, in particular during pregnancy.

According to the invention, dimethylpolysiloxane (dimeticone) was found to be effective in the treatment of constipation, without producing the usual side effects.

Commercial dimeticone-containing preparations are used to treat indications such as flatulence, sensation of repletion (bloating) and meteorism. Moreover, the use of dimeticone for treating inflammatory and ulcerous diseases of the esophagus, the stomach and the duodenum has been described.

In FR-5.549M, Therapiewoche 1975, Nr. 25, pp. 1509-1512, Med. J. Aust. Vol. 150, No. 11, 1989, p. 667, J. Indiana Med. Ass. Vol. 66, No. 12, 1973, pp. 1083-1084, DE-A-38 07 712 and Radiologie Vol. 30, No. 1, 1990 various compositions or therapeutic regimens for the treatment of constipation are disclosed which comprise in addition to a conventional laxative component either dimeticone or simethicone based on their deflatulent properties.

Dimethylpolysiloxane may be administered to a human or animal in the form of tablets, capsules, emulsions, suspensions and powders for oral administration or in combination with foodstuffs and dietary supplements. Such compositions may be administered to humans and animals in a safe and effective amount to elicit the desired result. The compositions used for this invention typically comprise an effective amount of dimethylpolysiloxane and a biologically acceptable carrier. Such carriers may be solid or liquid, such as, for example, highly dispersed silicon dioxide, cornstarch, lactose, saccharose, glucose, sucrose, water, water and fat emulsions such as glycerin stearate emulsions and flavoring agents. If a solid carrier is used, the dosage forms may be tablets, capsules or lozenges. Liquid dosage forms include soft gelatine capsules, syrup or liquid suspension. The dimeticone-containing compositions may be employed according to this invention in a conventional manner for the treatment and prevention of constipation. Their dosage levels may be chosen by those of ordinary skill in the art from the available methods and techniques. Specific dosage and treatment regimens will depend upon factors such as health status and the severity and course of constipation and disposition thereto.

In order that this invention be more fully understood, the following example is set forth. This example is for the purpose of illustration.

### Example

### Preparation of treatment composition

2 kg of dimethylpolysiloxane containing approximately 60 g of highly dispersed silicon dioxide was heated in a water bath to about 70°C. 1 kg of solid, finely triturated glycerin stearate (a mixture of mono- and diglycerin stearate) was then added thereto and the mixture was left in the water bath until the entire mass had melted. The mixture was then stirred to form an emulsion to which 3 liters of water were slowly added at a temperature of about 70°C. After further stirring and gradual cooling, a stable aqueous emulsion formed which had a stiff, lard-like composition at room temperature. This composition could now be finely dosed, admixed with an inert tabletting mass consisting of saccharose, glucose, lactose and flavoring agents, and made into tablets. It is also possible to dilute the resulting mass with water to prepare a liquid preparation for administration as drops.

| Application of treatment composition | |
|---|---|
| sex | female |
| age | 2 years |
| diagnosis | constipation |
| additional symptoms | tibia fracture (left side) resulting in a bed-ridden condition |
| duration of constipation | three days |
| concomitant symptoms | non-specific symptomatic pains |
| therapy | one teaspoon of the dimeticone suspension |
| result | complete evacuation of the feces (bowels) after two hours |

As the patient was required to remain immobilized, the situation after three further days was the same as before treatment. Again, about 40 drops (one teaspoon) of the dimeticone suspension was administered. After the suspension was allowed to act for 2 hours, it produced a complete evacuation of the bowels.

## Claims

1. Use of dimethylpolysiloxane as the sole laxative ingredient for the preparation of a pharmaceutical composition for treating constipation.

2. The use according to claim 1 wherein the dimethylpolysiloxane is used in combination with a highly dispersed silicon dioxide.

3. The use according to any one of claims 1 or 2 wherein the dimethylpolysiloxane is used in combination with a biologically acceptable emulsifier.

4. The use according to claim 3, wherein the emulsifier is glycerine stearate.

5. The use according to any one of claims 1 to 4 for treating constipation in a child, immobile or geriatric patient, or a pregnant woman.

## Patentansprüche

1. Verwendung von Dimethylpolysiloxan als einzigen abführend wirkenden Inhaltsstoff zur Herstellung eines Arzneimittels zur Behandlung von Verstopfung.

2. Verwendung nach Anspruch 1, wobei Dimethylpolysiloxan in Kombination mit hochdispergiertem Silicumdioxid verwendet wird.

3. Verwendung nach einem der Ansprüche 1 oder 2, wobei Dimethylpolysiloxan in Kombination mit einem biologisch verträglichen Emulgator verwendet wird.

4. Verwendung nach Anspruch 3, wobei der Emulgator Glycerinstearat ist.

5. Verwendung nach einem der Ansprüche 1 bis 4 zur Behandlung von Verstopfung bei einem Kind, einem bewegungsunfähigen oder alten Patienten oder einer schwangeren Frau.

## Revendications

1. Utilisation d'un diméthylpolysiloxane comme seul ingrédient laxatif pour la préparation d'une composition pharmaceutique pour le traitement de la constipation.

2. Utilisation selon la revendication 1, **caractérisée en ce que** le diméthylpolysiloxane est utilisé en combinaison avec un dioxyde de silicium fortement dispersé.

3. Utilisation selon l'une quelconque des revendications 1 ou 2, **caractérisée en ce que** le diméthylpolysiloxane est utilisé en combinaison avec un émulsifiant acceptable biologiquement.

4. Utilisation selon la revendication 3, **caractérisée en ce que** l'émulsifiant est le stéarate de glycérine.

5. Utilisation selon l'une quelconque des revendications 1 à 4 pour le traitement de la constipation chez un enfant, un patient invalide ou un vieillard ou une femme enceinte.
